# EUROPÄISCHE PATENTANMELDUNG

(11) **EP 0 717 021 A1**
(43) Veröffentlichungstag der Anmeldung: **19.06.1996**
(21) Anmeldenummer: 95119064.4
(22) Anmeldetag: 04.12.1995
(51) Int. Cl.: C07B 63/00, C07C 41/34, B01D 17/00

(54) **Methode zur Aufarbeitung eines tensidhaltigen Reaktionsgemisches**

(30) Priorität: 15.12.1994 DE 4444739
(71) Anmelder: BAYER AG, 51368 Leverkusen (DE)
(72) Erfinder: Wagner, Ortwin, D-56727 Mayen (DE); Schomäcker, Reinhard, Dr., D-51381 Leverkusen (DE)

(57) **Zusammenfassung**

Ein tensidhaltiges Reaktionsgemisch, bei dem jeweils ein Reaktionsprodukt als wäßrige Phase und ein weiteres Reaktionsprodukt als mit Wasser nicht mischbare Phase vorliegen und das in Form einer einphasigen Mikroemulsion erhalten wird, kann dadurch aufgearbeitet werden, daß man diese Mikroemulsion einer Temperaturänderung um 20 bis 50 K zu tieferen oder zu höheren Temperaturen unterwirft und dadurch eine hoch tensidhaltige Phase und eine weitgehend tensidfreie Phase bildet, die gebildete tensidfreie Phase abtrennt und jeweils eine abgetrennte wäßrige Phase durch die wäßrige Phase des ersten Reaktionspartners und eine abgetrennte organische Phase durch die organische Phase des zweiten Reaktionspartners ersetzt. Nach dem Ersatz der tensidfreien Phase wird das so behandelte Reaktionsgemisch einer zweiten Temperaturänderung um 10 bis 80 K in die zur ersten Temperaturänderung entgegengesetzten Richtung unterworfen und dadurch wiederum neben einer hoch tensidhaltigen eine tensidfreie Phase gebildet, die im obigen Sinne abgetrennt und ersetzt wird. Das danach vorliegende Gemisch kann der Reaktion erneut zugeführt werden.

## Beschreibung

Die vorliegende Erfindung betrifft die Aufarbeitung eines als einphasige Mikroemulsion anfallenden tensidhaltigen Reaktionsgemisches.

Bei vielen chemischen Reaktionen besitzen die Edukte und im allgemeinen auch die Produkte so unterschiedliche Löslichkeiten, daß sie weder in einem unpolaren organischen Lösungsmittel noch in Wasser gemeinsam gelöst werden können. Ein typisches Beispiel hierfür ist die Dehydrohalogenierung von 3,4-Dichlor-1-buten (wasserunlöslich) durch Natriumhydroxid, das in Form von wäßriger Natronlauge zugesetzt wird. Weitere Beispiele sind die Bildung von Nitrilen aus Alkylhalogeniden und anorganischen Cyaniden oder verschiedene Alkylierungsreaktionen in Gegenwart starker Basen (Nachr. Chem. Techn. Lab. 40, (1992), 1344). Den aufgezeigten Schwierigkeiten kann man beispielsweise wie folgt begegnen:
1. Man verwendet ein polares organisches Lösungsmittel (beispielsweise Methanol, Dimethylformamid, Acetonitril), das beide Edukte begrenzt löst. Die Reaktion verläuft dann in homogener Phase.
2. Man löst die Edukte in den von ihnen bevorzugten Lösungsmitteln und erzeugt durch ein Dispergierorgan (beispielsweise durch Rührer oder Düsen) eine große, innere Grenzfläche.
3. Man setzt den in ihren Lösungsmitteln gelösten Edukten einen Phasentransfer-Katalysator zu, der die Löslichkeit eines der beiden Reaktionspartner in dem Lösungsmittel des anderen erhöht.

Die aufgezeigten Methoden haben allerdings in der Regel einen Nachteil. So sind polare organische Lösungsmittel und Phasentransfer-Katalysatoren häufig umweltbelastend, weshalb man damit rechnen muß, daß ihr Einsatz zunehmend durch Umweltauflagen und die Notwendigkeit einer weitgehenden Aufarbeitung teurer wird und daher in der Regel Alternativen gesucht und gefunden werden müssen. Die Erzeugung einer großen Phasengrenzfläche durch Dispergieren kommt im allgemeinen nur für schnelle Reaktionen in Frage, da bei langsamen Reaktionen wegen der Notwendigkeit einer langen Aufrechterhaltung von Dispersionen viel Energie benötigt wird.

Eine neue Methode, die die Nachteile der obengenannten Methoden nicht besitzt ist die Erzeugung einer Mikroemulsion aus den in ihren bevorzugten Lösungsmitteln gelösten Edukten durch den Zusatz eines Tensids (J. Am. Chem. Soc. 113 (1991), 9621). Solche thermodynamisch stabilen Mikroemulsionen enthalten hochfeine Tröpfchen, deren Größe weit unterhalb der durch Dispergierverfahren erzeugten Tröpfchen liegt. Da die Mikroemulsion sich unter bestimmten Umständen spontan bildet, also aus eigener Kraft stabil ist, können hierin auch langsame Reaktionen besonders wirtschaftlich durchgeführt werden.

Während Phasentransfer-Katalysatoren und Dispergierverfahren bereits industriell eingesetzt werden, ist der Einsatz von Mikroemulsionsverfahren bisher über den Labormaßstab nicht hinausgelangt. Dies liegt daran, daß Mikroemulsionen nur in einem begrenzten Temperatur- und Konzentrationsbereich der Edukte stabil sind. Um daher für den industriellen Einsatz ausreichende Stabilitäten über einen größeren Temperaturbereich und eine Erhöhung der Eduktkonzentrationen zu erzielen, sind im allgemeinen erhöhte Tensidkonzentrationen notwendig. Solche erhöhten Tensidkonzentrationen erhöhen aber wiederum die Notwendigkeit einer Aufarbeitung solcher Mikroemulsionen, um das eingesetzte Tensid weitgehend zurückzugewinnen; diese Rückgewinnung verhindert zur gleichen Zeit Umweltbelastungen oder entsprechende Gegenmaßnahmen und andererseits Verunreinigungen des Produkts.

Es wurde nun gefunden, daß Reaktionsgemische in Form von Mikroemulsionen durch im weiteren näher beschriebene Temperaturänderungen in Mehrphasensysteme aufgetrennt werden können, von denen jeweils eine Phase hoch tensidhaltig ist, während eine weitere gebildete Phase weitgehend tensidfrei ist. Die tensidfreie Phase kann leicht abgetrennt werden und durch eine gleichartige (entweder wäßrige oder mit Wasser nicht mischbare organische) Phase im gesamten System ersetzt werden.

Die Erfindung betrifft eine Methode zur Aufarbeitung eines als Mikroemulsion vorliegenden Reaktionsgemisches, das durch Reaktion eines ersten Reaktionspartners, der als wäßrige Phase vorliegt, mit einem zweiten Reaktionspartner, der als mit Wasser nicht mischbare flüssige organische Phase vorliegt, in Gegenwart eines anionischen oder nicht-ionischen Tensids entstanden ist und das neben dem Tensid ein erstes Reaktionsprodukt, das in Wasser löslich ist, und ein zweites Reaktionsprodukt, das in der mit Wasser nicht mischbaren organischen Phase löslich ist, enthält und wobei die Mikroemulsion in Koexistenz mit wäßriger und mit organischer Phase vorliegen kann, die dadurch gekennzeichnet ist, daß man das zunächst als Mikroemulsion vorliegende Reaktionsgemisch
a) einer Temperaturänderung um 20 bis 50 K zu tieferen oder zu höheren Temperaturen unterwirft und dadurch eine hoch tensidhaltige Phase und eine weitgehend tensidfreie Phase erhält,
b) die weitgehend tensidfreie und Reaktionsprodukte enthaltende Phase abtrennt und jeweils eine abgetrennte wäßrige Phase durch die wäßrige Phase des ersten Reaktionspartners und eine abgetrennte organische Phase durch die organische Phase des zweiten Reaktionspartners ersetzt,
c) das von Schritt b) erhaltene Reaktionsgemisch einer zweiten Temperaturänderung um 10 bis 80 K in die zur ersten Temperaturänderung entgegengesetzten Richtung unterwirft und dadurch erneut neben einer hoch tensidhaltigen Phase eine weitgehend tensidfreie Phase erhält,
d) die bei c) gebildete weitgehend tensidfreie und Reaktionsprodukt enthaltende Phase abtrennt und in der unter b) genannten Art ersetzt und
e) das danach vorliegende reaktionsfähige Gemisch erneut der Reaktion zuführt.

Aus US 3.855.269 ist bekannt, eine Emulsion, die Adiponitril, Acrylnitril, gegebenenfalls weitere organische Verbindungen, anorganische Salze, Wasser und Tetraalkylammoniumsalze enthält, dadurch aufzuarbeiten, daß man das als Emulsion vorliegende und sich spontan trennende Reaktionsgemisch trennt und nur die organische Phase einer zweifachen Abkühlung unterwirft.

Aus JP 59/112 839 (1984) ist bekannt, ein quaternäres Ammoniumsalz, das als Katalysator bei der Herstellung von α-substituiertem Benzylnitril aus Benzylnitril mit Alkylhalogeniden eingesetzt worden war, mit Wasser zu extrahieren; das zu extrahierende Reaktionsgemisch lag jedoch nicht als Emulsion vor und enthielt ursprünglich auch kein Wasser.

Das erfindungsgemäß aufzuarbeitende Reaktionsgemisch liegt nach Abschluß der durchgeführten chemischen Reaktion in Form einer Mikroemulsion (ME) im Temperaturbereich von 0 bis 100°C, bevorzugt von 30 bis 80°C vor. Diese ME wird nun erfindungsgemäß nach a) zunächst einer Temperaturänderung unterworfen, bei der man die ME um 20 bis 50 K, ausgehend von der Temperatur, bei der die ME anfällt, zu tieferen oder zu höheren Temperaturen führt. Eine Änderung zu tiefen Temperaturen ist durch das Gefrieren des wäßrigen Anteils der ME begrenzt. Diese untere Temperatur liegt jedoch durch den Gehalt an Produkt bzw. nicht umgesetztem Edukt im Wasseranteil unterhalb von 0°C. Die Änderung zu höheren Temperaturen ist durch den Siedepunkt eines Anteils der ME begrenzt. Der Siedepunkt kann jedoch durch Anwendung von höheren Drücken zu höheren Temperaturen verschoben werden. Insofern wird die erfindungsgemäße Methode bei einem Druck von 1 bis 20 bar angewandt.

Bei der erfindungsgemäßen Temperaturänderung gemäß a) bildet sich neben einer hoch tensidhaltigen Phase eine weitgehend tensidfreie Phase. Diese weitgehend tensidfreie Phase kann entweder eine wäßrige Phase oder eine mit Wasser nicht mischbare organische Phase sein. Diese weitgehend tensidfreie Phase wird nun erfindungsgemäß nach b) mechanisch abgetrennt, beispielsweise durch Absitzenlassen in Trenngefäßen oder durch Abtrennung in Zentrifugen, und durch eine gleichartige Phase mit neuem Edukt ersetzt. Gleichartig heißt hierbei, daß eine weitgehend tensidfreie wäßrige Phase durch eine wäßrige Phase mit dem in Wasser löslichen Edukt ersetzt wird und daß eine weitgehend tensidfreie organische, mit Wasser nicht mischbare Phase durch das organische, mit Wasser nicht mischbare Edukt oder durch eine Lösung des organischen Edukts in einem mit Wasser nicht mischbaren Lösungsmittel ersetzt wird.

Erfindungsgemäß wird nach Abtrennung und Ersatz der weitgehend tensidfreien Phase in der oben beschriebenen Art eine zweite Temperaturänderung vorgenommen, die der ersten Temperaturänderung entgegengesetzt ist und die den Betrag von 10 bis 80 K, bevorzugt 20 bis 60 K, umfaßt. Auch hierbei werden die bereits genannten Grenzen der Eisbildung bzw. der Nichtüberschreitung des Siedepunkt eingehalten. Sodann wird nach d) auch die bei c) gebildete weitgehend tensidfreie Phase abgetrennt und durch eine gleichartige Phase ersetzt; das danach vorliegende Gemisch enthält nunmehr neben dem Tensid wieder beide Edukte und wird erneut der Reaktion zugeführt.

Die erfindungsgemäße Aufarbeitungsmethode kann demnach in folgenden Varianten ausgeführt werden:
(i) Das als ME anfallende ausreagierte Reaktionsgemisch wird um 20 bis 50 K erwärmt. Hierbei bildet sich neben der hoch tensidhaltigen Phase eine weitgehend tensidfreie Phase. Die Tatsache der Ausbildung einer weitgehend tensidfreien Phase ist abhängig von der Art der Reaktionspartner, von ihren Mengen, von der Art und Menge des eingesetzten Tensids, damit also ganz allgemein von der Zusammensetzung der ME, sowie von der Temperatur, bei der das ausreagierte Reaktionsgemisch anfällt und von der Temperatur auf die es durch die erfindungsgemäße Temperaturänderung gebracht wird. Ebenso abhängig von den genannten Parametern ist auch die Frage, ob die weitgehend tensidfreie Phase eine wäßrige oder eine mit Wasser nicht mischbare organische Phase ist. In analoger Weise wie oben geschildert, wird die entstandene weitgehend tensidfreie Phase abgetrennt und durch eine gleichartige Phase ersetzt. Danach wird dieses durch Temperaturerhöhung, Phasenabtrennung und Phasenersatz behandelte Reaktionsgemisch einer Temperaturerniedrigung um 10 bis 80 K unterworfen, wobei sich wiederum neben einer hoch tensidhaltigen Phase eine weitgehend tensidfreie Phase bildet. Die Art der tensidfreien Phase, die sich bei dieser zweiten Phasentrennung ausbildet, ist in ihrer Art (wäßrig oder organisch) entgegengesetzt zu der tensidfreien Phase, die sich bei der ersten Phasentrennung gebildet hat. Wenn sich also bei der zunächst durchgeführten Temperaturerhöhung eine tensidfreie organische Phase gebildet hatte, die ersetzt wurde, bildet sich bei der Temperaturerniedrigung eine tensidfreie wäßrige Phase, die ersetzt wird, und umgekehrt.
(ii) Das als ME anfallende ausreagierte Reaktionsgemisch wird in entgegengesetzter Weise zur Variante unter (i) zunächst um 20 bis 50 K abgekühlt, wobei sich in Abhängigkeit von der Zusammensetzung der ME und in Abhängigkeit der Temperaturen der angefallenen ME und ihrer Abkühlung neben der hoch tensidhaltigen Phase nur eine weitgehend tensidfreie Phase bildet, die im Sinne des oben Beschriebenen eine wäßrige oder eine organische, mit Wasser nicht mischbare Phase sein kann. Diese weitgehend tensidfreie Phase wird im Sinne des oben Beschriebenen abgetrennt und ersetzt, woraufhin das nunmehr neu entstandene Gemisch um 10 bis 80 K erwärmt wird. Nach dieser Erwärmung fällt erneut eine weitgehend tensidfreie Phase an, die in ihrer Art entgegengesetzt ist zur zunächst angefallenen weitgehend tensidfreien Phase. Auch diese wird im Sinne des oben Beschriebenen abgetrennt und ersetzt.

Nach beiden genannten Varianten (i) und (ii) erhält man schließlich ein Gemisch aus der hoch tensidhaltigen Phase und den beiden ersetzten Phasen (wäßrig bzw. organisch), die jedoch nunmehr wiederum Edukt für die vorgesehene Reaktion enthalten und so erneut der Reaktion zugeführt werden können. Hieraus wird durch geeignete Temperatureinstellung erneut eine ME gebildet.

Durch einfache Vorversuche kann vom Fachmann bestimmt werden, welche der Varianten (i) bzw. (ii) für ein vorliegendes Reaktionsgemisch zur Anwendung kommt. Infolge der zweifachen Temperaturänderung und der separaten Abtrennung der beiden weitgehend tensidfreien Phasen ist sichergestellt, daß die Reaktionsprodukte in den beiden abgetrennten Phasen (wäßrig bzw. organisch) nahezu vollständig abgetrennt und gewonnen werden.

Das erfindungsgemäße Verfahren ist gleichermaßen durchführbar, wenn im zu behandelnden Reaktionsgemisch die ME in Koexistenz mit der wäßrigen und mit der organischen Phase vorliegt. Dieser Fall tritt beispielsweise ein, wenn im unteren Teil der weiter unten genannten Tensidmenge gearbeitet wird und/oder Edukte und Produkte zwischen den in Koexistenz vorliegenden tensidfreien Phasen und der ME als Reaktionsphase wechseln. Hierdurch kann in wirtschaftlich und ökologisch günstiger Weise die Tensidmenge gesenkt werden. Bezogen auf das gesamte Reaktionsgemisch beträgt die Menge der ME 50 bis 100 Gew.-%, bevorzugt 70 bis 100 Gew.-%, die Menge der koexistierenden wäßrigen Phase 35 bis 0 Gew.-%, bevorzugt 25 bis 0 Gew.-% und die Menge der koexistierenden organischen Phase 35 bis 0 Gew.-%, bevorzugt 25 bis 0 Gew.-%.

Als Tenside zur Bildung der ME, die durch erfindungsgemäße Aufarbeitung gemeinsam mit frischen Edukten in die Reaktion zurückgeführt werden, kommen nichtionische und anionische Tenside in Frage. Diese Tenside stehen im Gegensatz zu Phasentransfer-Katalysatoren, bei denen es sich um kationische quaternäre Ammonium- oder Phosphoniumsalze handelt. Solche quaternären Salze sind ganz allgemein fischtoxisch, woraus sich die Notwendigkeit einer sorgfältigen Entsorgung ergibt. Sie sind ferner zersetzlich, etwa durch Abspaltung von Olefinen aus der quaternären Ammonium- bzw. Phosphoniumgruppe.

Nicht ionische Tenside für erfindungsgemäß aufzuarbeitende Reaktionsgemische sind Ethoxylate, Propoxylate und gemischte Ethoxylat-propoxylate von Alkoholen, Phenolen, Alkylphenolen, Carbonsäuren oder Carbonsäureamiden sowie langkettigen Fettaminen, bei denen das zu alkoxylierende Grundmolekül 6 bis 30 C-Atome hat und der Alkoxylierungsgrad 4 bis 40 Ethylenoxid- bzw. Propylenoxid-Gruppen umfaßt. In bevorzugter Weise handelt es sich hierbei um alkoxylierte Alkohole oder Phenole, die in besonders bevorzugter Weise durch eine C₄-C₁₆-Alkylkette substituiert sind. Die Alkylkette kann hierbei geradkettig oder verzweigt sein.

Anionische Tenside für das erfindungsgemäß aufzuarbeitende Reaktionsgemisch sind beispielsweise: Alkylsulfate, Alkylsulfonate und Alkylcarboxylate mit Alkylketten mit 4 bis 18 C-Atomen. Als Kationen können diese Tenside Alkali- oder Erdalkaliionen, sowie Ammonium-Ionen enthalten.

Die erfindungsgemäße Methode wird in bevorzugter Weise auf solche Reaktionsgemische, die als ME vorliegen, angewandt, die mit Hilfe nichtionischer Tenside gebildet wurden. Hierbei kann mit einem oder mit mehreren der genannten nichtionischen Tenside gearbeitet werden. Im allgemeinen fallen nichtionische Tenside ohnehin als Gemisch verschieden hoch alkoxylierter Grundmoleküle an, so daß der angegebene Alkoxylierungsgrad einen Mittelwert innerhalb einer dem Fachmann bekannten Bandbreite darstellt. In vielen Fällen wurde beobachtet, daß beim Einsatz nichtionischer Tenside vorteilhaft zunächst eine Abkühlung im Sinne der obigen Variante (ii) gearbeitet wird. Hierbei bildet sich bei Variante (ii) eine weitgehend tensidfreie organische, mit Wasser nicht mischbare Produktphase, die abgetrennt und durch die organische Eduktphase ersetzt wird, während gleichzeitig eine hoch tensidhaltige wäßrige Phase entsteht.

Die Menge an Tensid, die in dem als ME anfallenden ausreagierten Reaktionsgemisch enthalten ist, beträgt 2 bis 50 Gew.-%, bevorzugt 5 bis 30 Gew.-%, besonders bevorzugt 7 bis 20 Gew.-%, bezogen auf das Gesamtgewicht des Reaktionsgemisches.

Die erfindungsgemäße Aufarbeitungsmethode ist auf als ME vorliegende ausreagierte Reaktionsgemische anwendbar, in denen eine Vielzahl von Reaktionen abgelaufen sein können, bei denen Reaktionspartner aufeinander getroffen sind, von denen einer vorwiegend in wäßriger Phase vorlag und der andere als mit Wasser nicht mischbare organische Phase. Der als organische Phase vorliegende Reaktionspartner (Edukt) kann den organischen Anteil der ME allein bilden, wenn er in dem hier in Frage kommenden Temperaturbereich flüssig ist. Für den Fall, daß der in Wasser nicht lösliche organische Reaktionspartner gasförmig oder fest ist, wird er in Form einer Lösung in einem organischen, mit Wasser nicht mischbaren Lösungsmittel eingesetzt. Lösungsmittel, die hierfür in Frage kommen sind beispielsweise: aliphatische C₄-C₂₀- oder aromatische C₆-C₁₀-Kohlenwasserstoffe, die gegebenenfalls durch Halogen, Nitro oder Estergruppen substituiert sein können und dem Fachmann als typische organische Lösungsmittel geläufig sind.

Reaktionen die zu erfindungsgemäß aufzuarbeitenden ME führen, sind beispielsweise die folgenden:
- die Veretherung,
- die Dehydrohalogenierung von Halogenkohlenwasserstoffen durch wäßriges Alkalihydroxid, beispielsweise die Dehydrochlorierung von 3,4-Dichlor-1-buten mit NaOH,
- die Bildung von Nitrilen aus Alkylhalogeniden und anorganischen Cyaniden,
- die Alkylierung von Alkoholen, Phenolen, Carbonsäuren, Aminen oder Amiden mit Hilfe von Alkylhalogeniden in Gegenwart starker Basen,
- die α-Alkylierung von aktivierten Verbindungen mit Hilfe von Halogenalkylen, z.B. die Bildung α-alkylierter Nitrile aus Nitrilen und Halogenalkylen,
- Hydrolysen von Estern in Gegenwart starker Basen,
- Oxidationen von Organika mit Bleichlauge,
- Oxidationen von Organika mit Sauerstoff in Gegenwart von wasserlöslichen Katalysatoren,
- Reduktionen mit wasserlöslichen Katalysatoren,
- Oxidationen von Organika mit H₂O₂ in Gegenwart wasserlöslicher Katalysatoren,
- Oxidationen mit wäßriger Cer(IV)sulfat-Lösungen,
- saure oder alkalische Abspaltungen von Schutzgruppen.

Bevorzugt hiervon sind als Reaktionen:
- die Dehydrohalogenierung von Halogenkohlenwasserstoffen
- die Bildung von Nitrilen aus Alkylhalogeniden und anorganischen Cyaniden,
- die Alkylierung von Alkoholen, Phenolen, Carbonsäuren, Aminen oder Amiden,
- die α-Alkylierung von aktivierten Verbindungen oder
- die Hydrolyse von Estern.

Anhand der O-Alkylierung von Phenolen mit einem Alkylhalogenid in Gegenwart von Natriumhydroxid sei ein solches Verfahren wie folgt formuliert:

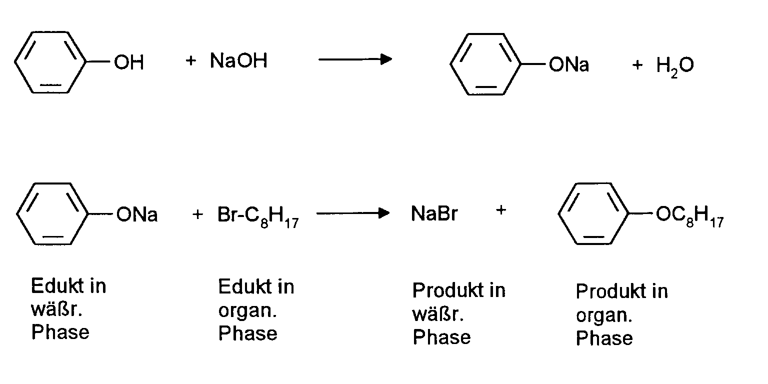

### Beispiel

Ein Reaktionsgemisch in Form einer Mikroemulsion (ME), in welchem n-Octylbromid und Natriumphenolat, gelöst in Wasser, bei 85°C umgesetzt worden waren, bestand aus 20 Gew.-% Phenyl-octylether, 60 Gew.-% 1N-NaBr-Lösung und 20 Gew.-% des Tensids Triton X® (Octylphenol + 10 Ethylenoxid-Einheiten). Diese ME wurde auf 50°C abgekühlt. Dabei bildeten sich zwei Phasen. Hiervon war die wäßrige Phase hoch tensidhaltig, während die organische, mit Wasser nicht mischbare Phase im wesentlichen aus Phenyl-octylether bestand. Die organische Phase wurde abgetrennt und durch das gleiche Gewicht an Octylbromid ersetzt. Das durch diesen Ersatz entstandene neue Gemisch wurde auf 70°C erwärmt. Hierbei bildeten sich erneut zwei Phasen. Die hoch tensidhaltige Phase war nunmehr die organische, mit Wasser nicht mischbare Phase, die den Phenyl-octylether enthielt. Die weitgehend tensidfreie wäßrige NaBr-Lösung wurde abgetrennt und durch das gleiche Gewicht an 1N-Natriumphenolat-Lösung in Wasser ersetzt. Das bei diesem zweiten Ersatz entstandene Reaktionsgemisch wurde erneut der Umsetzung zur Bildung des Phenyl-octylethers zugeführt. Der Verlust an Tensid betrug ca. 5 % des ursprünglich eingesetzten.

## Patentansprüche

1. Methode zur Aufarbeitung eines als Mikroemulsion vorliegenden Reaktionsgemisches, das durch Reaktion eines ersten Reaktionspartners, der als wäßrige Lösung vorliegt, mit einem zweiten Reaktionspartner, der als mit Wasser nicht mischbare flüssige organische Phase vorliegt, in Gegenwart eines anionischen oder nichtionischen Tensids entstanden ist und das neben dem Tensid ein erstes Reaktionsprodukt, das in Wasser löslich ist, und ein zweites Reaktionsprodukt, das in der mit Wasser nicht mischbaren organischen Phase löslich ist, enthält und wobei die Mikroemulsion in Koexistenz mit wäßriger und mit organischer Phase vorliegen kann, dadurch gekennzeichnet, daß man das zunächst als Mikroemulsion vorliegende Reaktionsgemisch
a) einer Temperaturänderung um 20 bis 50 K zu tieferen oder zu höheren Temperaturen unterwirft und dadurch eine hoch tensidhaltige Phase und eine weitgehend tensidfreie Phase erhält,
b) die weitgehend tensidfreie und Reaktionsprodukte enthaltende Phase abtrennt und jeweils eine abgetrennte wäßrige Phase durch die wäßrige Phase des ersten Reaktionspartners und eine abgetrennte organische Phase durch die organische Phase des zweiten Reaktionspartners ersetzt,
c) das von Schritt b) erhaltene Reaktionsgemisch einer zweiten Temperaturänderung um 10 bis 80 K in die zur ersten Temperaturänderung entgegengesetzten Richtung unterwirft und dadurch erneut neben einer hoch tensidhaltigen Phase eine weitgehend tensidfreie Phase erhält,
d) die bei c) gebildete weitgehend tensidfreie und Reaktionsprodukt enthaltende Phase abtrennt und in der unter b) genannten Art ersetzt und
e) das danach vorliegende reaktionsfähige Gemisch erneut der Reaktion zuführt.

2. Methode nach Anspruch 1, dadurch gekennzeichnet, daß ein nichtionisches Tensid vorliegt und die Temperaturänderung unter a) zu tieferen Temperaturen hin vorgenommen wird, wodurch eine hochtensidhaltige wäßrige Phase entsteht.

3. Methode nach Anspruch 2, dadurch gekennzeichnet, daß ein nichtionisches Tensid aus der Gruppe von alkoxylierten Phenolen und alkoxylierten C₄-C₁₆-Alkyl-phenolen eingesetzt wird.

4. Methode nach Anspruch 1, dadurch gekennzeichnet, daß das Reaktionsgemisch 2 bis 50 Gew.-%, bevorzugt 5 bis 30 Gew.-%, besonders bevorzugt 7 bis 20 Gew.-% Tensid, bezogen auf das Gesamtgewicht des Reaktionsgemisches, enthält.

5. Methode nach Anspruch 1, dadurch gekennzeichnet, daß die Reaktion, die zu dem zu behandelnden Reaktionsgemisch führt, ausgewählt ist aus folgenden Reaktionen:
- der Veretherung,
- der Dehydrohalogenierung von Halogenkohlenwasserstoffen durch wäßriges Alkalihydroxid, beispielsweise der Dehydrochlorierung von 3,4-Dichlor-1-buten mit NaOH,
- der Bildung von Nitrilen aus Alkylhalogeniden und anorganischen Cyaniden,
- der Alkylierung von Alkoholen, Phenolen, Carbonsäuren, Aminen oder Amiden mit Hilfe von Alkylhalogeniden in Gegenwart starker Basen,
- der α-Alkylierung von aktivierten Verbindungen mit Hilfe von Halogenalkylen, z.B. der Bildung α-alkylierter Nitrile aus Nitrilen und Halogenalkylen,
- Hydrolysen von Estern in Gegenwart starker Basen,
- Oxidationen von Organika mit Bleichlauge,
- Oxidationen von Organika mit Sauerstoff in Gegenwart von wasserlöslichen Katalysatoren,
- Reduktionen mit wasserlöslichen Katalysatoren,
- Oxidationen von Organika mit H₂O₂ in Gegenwart wasserlöslicher Katalysatoren,
- Oxidationen mit wäßriger Cer(IV)sulfat-Lösungen,
- saure oder alkalische Abspaltungen von Schutzgruppen.

6. Methode nach Anspruch 5, dadurch gekennzeichnet, daß als Reaktion
- die Dehydrohalogenierung von Halogenkohlenwasserstoffen,
- die Bildung von Nitrilen aus Alkylhalogeniden und anorganischen Cyaniden,
- die Alkylierung von Alkoholen, Phenolen, Carbonsäuren, Aminen oder Amiden,
- die α-Alkylierung von aktivierten Verbindungen oder
- die Hydrolyse von Estern durchgeführt wird.

7. Methode nach Anspruch 1, dadurch gekennzeichnet, daß die abzutrennende organische Phase für den Fall eines flüssigen Reaktionspartners frei von zusätzlichen Lösungsmitteln ist.

8. Verfahren nach Anspruch 1, dadurch gekennzeichnet, daß für den Fall der Koexistenz von Mikroemulsion und wäßriger und organischer Phase die Menge der Mikroemulsion 50 bis 100 Gew.-%, bevorzugt 70 bis 100 Gew.-%, des gesamten Reaktionsgemisches, die Menge der koexistierenden wäßrige Phase 35 bis 0 Gew.-%, bevorzugt 25 bis 0 Gew.-%, des gesamten Reaktionsgemisches und die Menge der koexistierenden organischen 35 bis 0 Gew.-%, bevorzugt 25 bis 0 Gew.-%, des gesamten Reaktionsgemisches darstellt.
